# EUROPEAN PATENT APPLICATION

(11) **EP 1 685 823 A1**
(43) Date of publication of application: **02.08.2006**
(21) Application number: 04818536.7
(22) Date of filing: 15.11.2004
(51) Int. Cl.: A61K 8/18, A61K 8/99, A61P 17/00, A61P 17/16

(54) **COSMETIC COMPOSITION**

(30) Priority: 17.11.2003 JP 2003386890
(71) Applicant: GEO Corporation, Kasuga-shi, Aichi 486-0918 (JP)
(72) Inventor: KAWASAKI, Kiwamu, Nagoya-shi, Aichi 464-0005 (JP)
(74) Representative: Schnappauf, Georg
(86) International application number: PCT/JP2004/016939
(87) International publication number: WO 2005/046624

(57) **Abstract**

A cosmetic composition effective in skin lightening or skin rejuvenation is provided. The cosmetic composition is **characterized by** containing sodium ions, potassium ions, calcium ions, magnesium ions, chlorine ions and HCO₃ ions in a molar concentration proportion equal to that in the extracellular fluid of mammals and further containing glucose in a concentration of 0.05 to 0.15 mol/L and sodium glutamate in a concentration of 0.01 to 0.03 mol/L.

## Description

### Technical Field

The present invention relates to a cosmetic composition, particularly, a cosmetic composition effective in skin lightening and skin rejuvenation.

### Background Art

Recently, the various efficacies of antioxidants have been taken up and come into the spotlight. For searching such antioxidants, various substances including ascorbic acid, α-tocopherol, polyphenols and lignins have been studied. Among such various substances, there are some substances including glutathione which exhibit strong antioxidant effect and therefore are expected to have pharmacological effect.

Many antioxidants, however, function also as oxidative substances depending on conditions such as concentration or pH. It has been particularly found that ascorbic acid acts as an oxidative substance in a low concentration such as 0.01 M (for example, see FEBS Letters, 405, 186-190 (1997)).

It has been found that an aqueous solution containing salts and saccharides extends the shelf life of fresh food, and said effect results from the antioxidant action of the aqueous solution (for example, see JP-A 2001-346560; Japanese Society of Animal Science, the 100th convention, proceedings of lecture, page 165 (2002); and Antioxidant Biofactor (AOB) Researching Organization, the second meeting, abstract, page 14 (2002)). It has been also found that such an aqueous solution can be used as cosmetics useful for preventing dry skin or alleviating inflammation excited by acne or the like, and used as medicine effective in preventing or treating diseases of skin or mucous membranes (for example, see JP-A 2001-348321, and JP-A 2002-308783).

### Disclosure of Invention

### Problem to be solved by the Invention

The present inventor intensively studied with the purpose of obtaining an antioxidant agent which acts more safely and effectively on the human body, has only antioxidant effect and does not act as an oxidative substance. As a result, the present inventor found that an aqueous solution containing salts and saccharides, particularly glucose, answers the purpose and is useful as a cosmetic composition effective in skin lightening and skin rejuvenation, and then completed the present invention.

### Means for Solving the Problem

That is to say, the present invention provides:
(1) a cosmetic composition which comprises sodium ions, potassium ions, calcium ions, magnesium ions, chlorine ions and HCO₃ ions in a molar concentration proportion equal to that in the extracellular fluid of mammals, and further comprises glucose in a concentration of 0.05 to 0.15 mol/L and sodium glutamate in a concentration of 0.01 to 0.03 mol/L;
(2) the composition according to the above (1), which is a skin-lightening cosmetic;
(3) the composition according to the above (1), which is a cosmetic for skin rejuvenation; and
(4) the composition according to the above (1), which is an aqueous solution.

### Effect of the Invention

Arbutin, kojic acid, retinol and the like are known to have skin-lightening effect resulting from their inhibitory action on the tyrosinase activity which is a trigger for melanin production. In contrast, the skin-lightening effect of the cosmetic composition of the present invention results from decomposing melanin granules produced in cells while increasing the vitality of the cells themselves. Thus, the mechanism of the skin-lightening effect differs between the cosmetic composition of the present invention and conventional cosmetics.

The cosmetic composition of the present invention may also have skin-rejuvenation effect due to an increase in the vitality of cells themselves.

Uptake of the main components of the cosmetic composition of the present invention was confirmed in human normal melanocytes, which are melanin-producing cells. Therefore, the cosmetic composition of the present invention can be absorbed percutaneously without hindrance.

### Best Mode for Carrying Out the Invention

The cosmetic composition of the present invention is an aqueous liquid agent, particularly aqueous solution, containing sodium glutamate, the above-mentioned salts and glucose as solutes.

The salts include cosmetically acceptable compounds which can be sources of sodium ions, potassium ions, calcium ions or magnesium ions as cations, or chlorine ions or HCO₃ ions as anions, and these compounds may be used as an appropriate mixture. The salts also include sea salts, raw salts and the like. Above all, use of calcium lactate as a calcium ion source promotes positive uptake of calcium ions into cells, so that calcium lactate is preferable.

The salts are contained in the composition of the present invention so that each ion can be present in the composition in a molar concentration proportion equal to that in the normal extracellular fluid of mammals. Generally, the molar concentration proportion of sodium ions : potassium ions : calcium ions : magnesium ions in the composition is 0.130 to 0.150 mol/L : 0.001 to 0.007 mol/L : 0.002 to 0.005 mol/L : 0.001 to 0.003 mol/L, and the molar concentration proportion of chlorine ions : HCO₃ ions in the composition is 0.080 to 0.150 mol/L : 0.02 to 0.04 mol/L. Typically, for example, the molar concentration proportion of Na : K : Ca : Mg ions in the composition is 0.14 mol/L : 0.004 mol/L : 0.0025 mol/L : 0.0015 mol/L, and the molar concentration proportion of Cl : HCO₃ ions in the composition is 0.1 mol/L : 0.027 mol/L.

In adjusting the molar concentration, for example, when 0.14 mol/L of sodium chloride is added, the concentration of chlorine ions is also 0.14 mol/L. Thus the above-mentioned molar concentration proportion can not be accomplished by simple blending. In the present invention, Na ions contained in sodium glutamate supplement the molar concentration of Na ions in a solution, and thereby the solution can be adjusted so as to have the above-mentioned proportion. The content of sodium glutamate in the composition is selected from the range of 0.01 to 0.03 mol/L.

Although the supplementation of Na ions can be also accomplished by an addition of other Na-containing compounds, sodium glutamate further promotes uptake of glucose into cells and then enhances the cell activity.

In the present invention, glucose, which has only reducing action, does not act as an oxidative substance and is safe, is used as essential saccharides. The content of glucose in the composition is selected from the range of 0.05 to 0.15 mol/L.

Other saccharides including, for example, monosaccharides such as galactose, mannose, fructose, xylose and arabinose, disaccharides such as maltose, lactose, sucrose and trehalose, and water-soluble oligosaccharide may be used in combination with glucose if desired.

In the present invention, in addition to the above solutes, mucoprotein or mucopolysaccharides such as collagen, gelatin, hyaluronic acid or chondroitin sulfuric acid may be added within such range that the total molar concentration of solutes is less than 0.54 mol/L. Within such concentration range of solutes, other active ingredients such as Japanese pepper extract, citrus peel extract, cinnamon extract, Angelicae Radix (Toki) extract, star anise extract, sophorae radix extract, licorice extract or Coicis semen (Yokuinin) extract, or additives such as inositol, niacin, niacinamide, glucuronic acid or glucosamine may be further added, if necessary.

The cosmetic composition of the present invention can be produced in the dosage form of an aqueous liquid agent, preferably an aqueous solution, by a per se known method comprising mixing and dissolving the desirable components and the like. By simply dissolving the desirable components in purified water, a solution with pH 7.35 to 7.45, which is suitable for the human body, can be obtained without particularly performing pH controlling.

Each component of the cosmetic composition of the present invention is extremely safe. Thus, for example, the cosmetics of the present invention can be applied to skin as appropriate and thereby can be used for skin lightening, skin rejuvenation and the like.

Hereinafter, the present invention is explained in detail with Examples, but is not limited thereto.

### Example 1

According to the following formulation (in regard to salts, the concentration of ions was inside parentheses), all ingredients were dissolved in purified water to obtain a cosmetic composition of the present invention in the form of an aqueous solution.

| | |
|---|---|
| NaCl | 5.61 g/L (0.0960 mol/L) |
| KCl | 0.30 g/L (0.0040 mol/L) |
| Ca(C₃H₅O₃)₂ | 0.77 g/L (0.0025 mol/L) |
| MgSO₄ | 0.18 g/L (0.0015 mol/L) |
| NaHCO₃ | 2.27 g/L (0.0270 mol/L) |
| sodium glutamate | 2.88 g/L (0.0170 mol/L) |
| glucose | 20.00 g/L (0.1110 mol/L) |
| trehalose | 10.00 g/L (0.0292 mol/L) |
| maltose | 5.00 g/L (0.0146 mol/L) |

### Example 2

### Test for skin lightening

### (1) Observation with electron microscope

Normal human neonatal preputial epidermal melanocytes (NHEM, KM4009) were cultured in HGMS-added Medium 154 (manufactured by Kurabo Industries Ltd.) at 37°C for 8 days under 5% CO₂-containing wet atmosphere. As a control group, the cells were incubated in HGMS-added Medium 154, and as a test group, the cells were incubated in HGMS-added Medium 154 to which all ingredients described in Example 1 were added at the concentrations described in Example 1 (1.0-time amount addition). After 8 days, the cells were collected. The cells were prefixed with glutaraldehyde, embedded and then observed with a JEOL JEM-2000EX electron microscope.

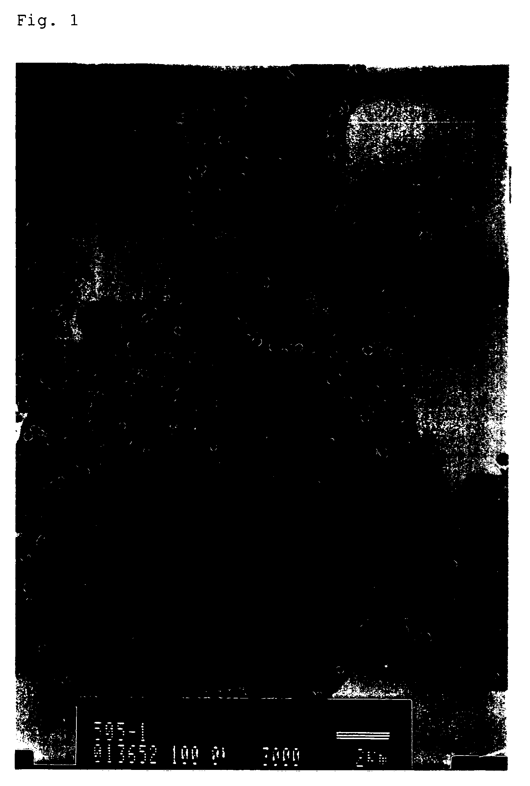

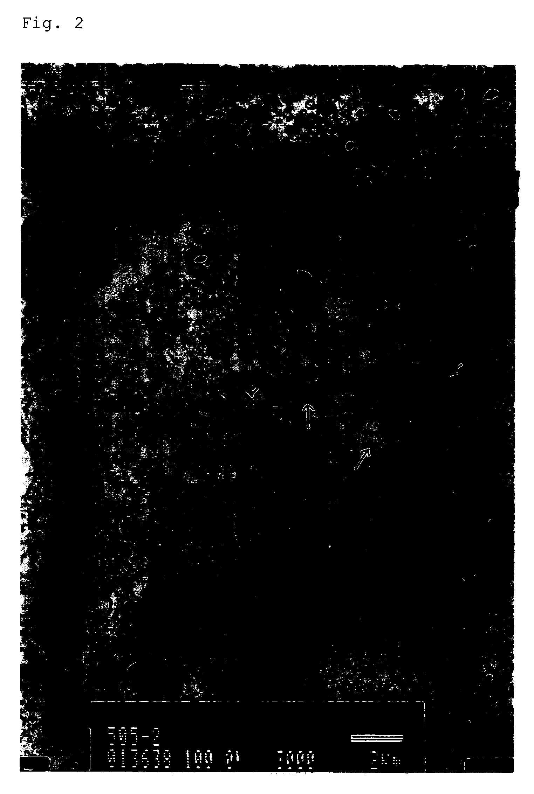

Fig. 1 and Fig. 2 show an approximately 5300-times magnification of the cells in the control group and the test group, respectively. As shown in Fig. 1, in the cells of the control group, many melanin granules (black elliptic grains) were observed, and the separation of cytoplasm filled with melanin granules was observed. In the cells of the test group, decomposition of melanin granules in the lysosome was observed as indicated by arrows.

### (2) Measurement of melanin amount

NHEM cells were cultured in the same manner as in the above-mentioned (1) for 8 days, in HGMS-added Medium 154 as a control group, in HGMS-added Medium 154 to which the ingredients described in Example 1 were added at the concentrations 0.5 times (0.5-times amount addition group), 1.0 time (1.0-time amount addition group) and 1.5 times (1.5-times amount addition group) the concentration described in Example 1 as test groups, that is, in the total 4 groups. Samples were collected on the second day, the fourth day and the eighth day and the melanin absorbance (475 nm) per protein amount was measured for each sample. Five samples were collected per each group and their average ± standard deviation was regarded as the measured value.

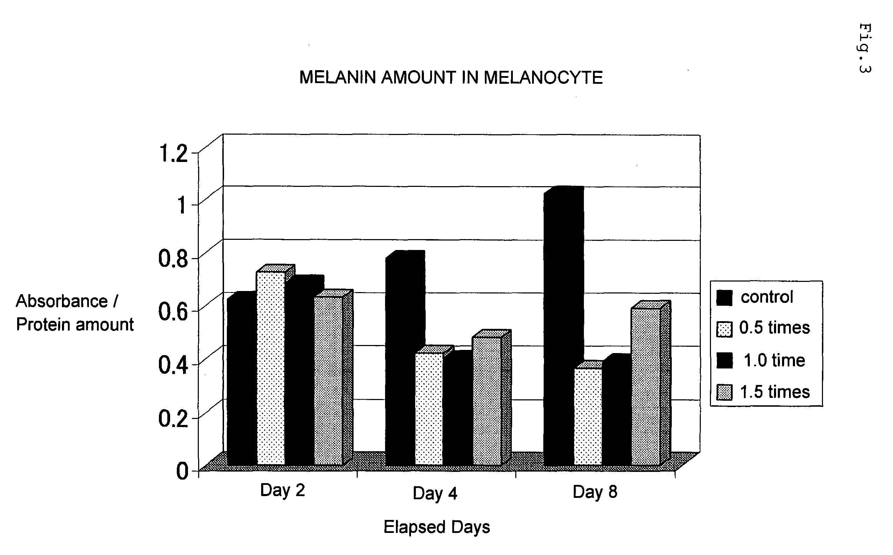

The results are shown in Table 1 and Fig. 3.

**Table 1**

| | Day 2 | Day 4 | Day 8 |
|---|---|---|---|
| control group | 0.62±0.06 | 0.78±0.13 | 1.02±0.31 |
| 0.5-times amount addition group | 0.73±0.17 | 0.42±0.15 | 0.37±0.26 |
| 1.0-time amount addition group | 0.68±0.08 | 0.40±0.10 | 0.39±0.10 |
| 1.5-times amount addition group | 0.63±0.05 | 0.48±0.17 | 0.59±0.03 |

In contrast to the control group, decrease in the melanin amount was observed with time in the 0.5-times amount addition group and the 1.0-time amount addition group (statistical significant difference was confirmed between the control group, and 0.5-times amount addition group and 1.0-time amount addition group by Fisher's PLSD method).

Thus, a cosmetic composition of the present invention decomposes melanin granules and thereby exerts skin lightening effect.

### Example 3

### Test for skin rejuvenation (activation)

NHEM cells were cultured in the same manner as in Example 2 (2), and activation effects of additives on the cells were examined by determining an increase of formazan production in mitochondria with MTT analysis. Five samples per each group were collected and their average ± standard deviation was regarded as the measured value.

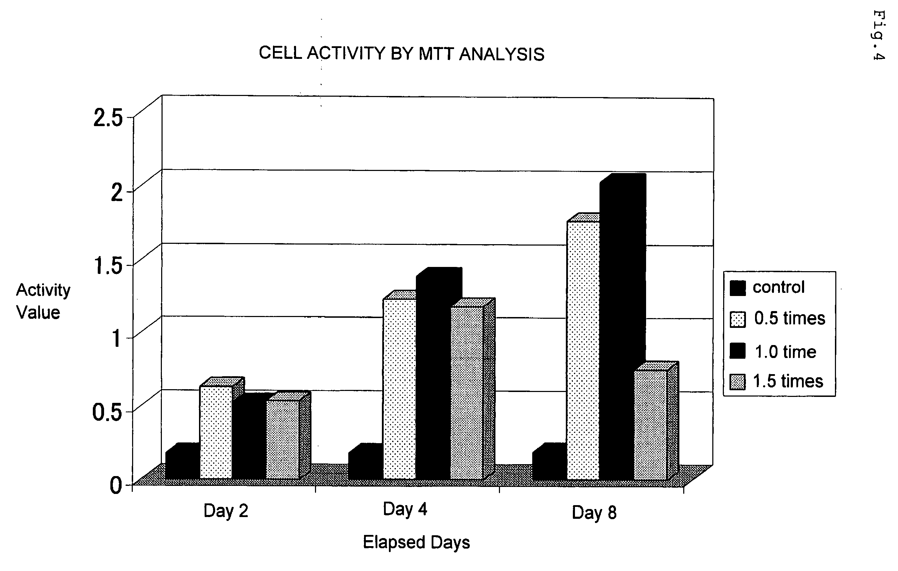

The results are shown in Table 2 and Fig. 4.

**Table 2**

| | Day 2 | Day 4 | Day 8 |
|---|---|---|---|
| control group | 0.18±0.006 | 0.18±0.02 | 0.19±0.008 |
| 0.5-times amount addition group | 0.63±0.042 | 1.23±0.08 | 1.77±0.66 |
| 1.0-time amount addition group | 0.52±0.04 | 1.39±0.04 | 2.03±0.05 |
| 1.5-times amount addition group | 0.54±0.02 | 1.18±0.06 | 0.75±0.05 |

As compared with the control group, the addition groups showed more increased formazan production with time (statistical significant difference was confirmed between a control group and all of the addition groups by Fisher's PLSD method). However, in the 1.5-times amount addition group, the amount of formazan production started to decrease from the sixth day and decreased obviously on the eighth day.

Thus, it was found that a cosmetic composition of the present invention enhanced the activity of cells.

### Industrial Applicability

As described above, the cosmetic composition of the present invention exerts excellent effects of skin lightening and skin rejuvenation.

### Brief Description of Drawings

Fig. 1 shows cells of the control group in Example 2.
Fig. 2 shows cells of the test group in Example 2.
Fig. 3 is a graph showing change with time of melanin measured values in Example 2.
Fig. 4 is a graph showing change with time of MTT analysis values in Example 3.

## Claims

1. A cosmetic composition which comprises sodium ions, potassium ions, calcium ions, magnesium ions, chlorine ions and HCO₃ ions in a molar concentration proportion equal to that in the extracellular fluid of mammals, and further comprises glucose in a concentration of 0.05 to 0.15 mol/L and sodium glutamate in a concentration of 0.01 to 0.03 mol/L.

2. The composition according to Claim 1, which is a skin-lightening cosmetic.

3. The composition according to Claim 1, which is a cosmetic for skin rejuvenation.

4. The composition according to Claim 1, which is an aqueous solution.
